Europäisches Patentamt

European Patent Office

Office européen des brevets

(11) Publication number: **0 160 239 B1**

(12) **EUROPEAN PATENT SPECIFICATION**

(45) Date of publication of patent specification: **14.08.91**  (51) Int. Cl.⁵: **G01N 33/72**, G01N 33/52

(21) Application number: **85104304.2**

(22) Date of filing: **10.04.85**

(54) Test piece for detecting bilirubin.

(30) Priority: **02.05.84 JP 87859/84**

(43) Date of publication of application:
**06.11.85 Bulletin 85/45**

(45) Publication of the grant of the patent:
**14.08.91 Bulletin 91/33**

(84) Designated Contracting States:
**BE DE FR IT**

(56) References cited:
**EP-A- 0 037 056**
**EP-A- 0 103 958**
**DE-A- 2 007 013**
**DE-A- 2 623 087**
**US-A- 3 411 887**

(73) Proprietor: **TERUMO KABUSHIKI KAISHA trading as TERUMO CORPORATION**
**44-1, 2-chome, Hatagaya Shibuya-Ku**
**Tokyo 151(JP)**

(72) Inventor: **Kaminagayoshi, Satoshi**
**24-19, Senbonkita 2-chome Nishinari-ku**
**Osaka-shi Osaka(JP)**

(74) Representative: **Henkel, Feiler, Hänzel & Partner**
**Möhlstrasse 37**
**W-8000 München 80(DE)**

EP 0 160 239 B1

## Description

Field of the Invention:

This invention relates to a test piece which is not susceptible of the influence of any reducing substance, and particularly to an improved test piece for detecting bilirubin in urine and blood, in a test piece for detecting of bilirubin by virtue of the diazo coupling reaction.

Description of Prior Art:

Recently, rapid diagnostic agents are gaining notably in significance in medical practice and in clinical tests. Rapid diagnostic agents having absorbent carrier which, in most cases, is paper is impregnated with a chemical agent necessary for the reaction of detection and immersed in a given body fluid specimen, it undergoes a color reaction if the substance under detection is present in the specimen. A rapid diagnostic agent for the detection of bilirubin is one example of the rapid diagnostic agents which are now gaining greatly in significance in medical diagnosis. Bilirubin in known to be formed from hemoglobin while red blood cells are undergoing normal and abnormal destruction by the reticulo-endothelial system. The detection and determination of bilirubin, therefore, are important for the purpose of discerning morbid conditions liable to induce excessive destruction of red blood cells and obstacles in the mechanism for excretion of bile. They are frequently resorted to in clinical tests.

The test under discussion is based on the following principle. In the determination of bilirubin, the bilirubin (coupling agent) is caused to react with a diazo compound to form an azo compound possessing a red chromophore, azo group $-N = N-$. The color of the azo compound serves as a criterion for the amount of bilirubin because the density of the color has a sufficient correlation with the concentration to bilirubin.

Recently-processed foodstuffs and refreshing beverages contain ascorbic acid in large amount as vitamin C and generous ingestion of vitamin preparations for the preservation of health is in vogue. Thus, such vitamins are richly contained in blood and excess water-soluble vitamins are excreted from the human body in urine. When the aforementioned diagnostic agent is used in the test of urine or blood, therefore, there is entailed the problem that the strong reducing power exhibited by the ascorbic acid present in the urine or blood will interfere with the diazo coupling reaction and impede ample coloration.

DE-A-2 623 087 discloses a test strip for detecting bilirubin by virtue of the diazo coupling reaction. However, this document does not teach the incorporation of any oxidizing agent for preventing influence of ascorbic acid in said body fluid.

EP-A-0 037056 discloses a test strip for the detection of bilirubin in body fluids. The disturbing effect of any reducing agent, mainly ascorbic acid, is eliminated by addition of a soluble iodate compound. This document does not disclose the use of sodium periodate.

US-A-3 411 887 discloses a test composition for detecting specific constituents in body fluids, which contain interfering reducing substances such as ascorbic acid. The known test composition comprises an enzymatic detecting system which releases peroxide and a trapping system for oxidizing the interfering reducing substance. Said trapping system comprises an ionizable heavy metal compound.

EP-A-0 103 958 discloses a solid support "dip-stick" immunoassay wherein a peroxidase catalyses the formation of a dye. Ascorbate, which interferes with the assay, is reduced or eliminated by impregnating the support with periodate.

DE-A-20 07 013 discloses reactants which form a diazo compound, on a diagnostic means.

An object of the present invention, therefore, is to provide a novel test piece which provides effective detection of bilirubin by virtue of the diazo coupling reaction without being affected by any reducing substance.

Another object of this invention is to provide a test piece which excels in stability appropriate for the detection of bilirubin in blood and urine.

## SUMMARY OF THE INVENTION

The objects described above are attained by a test piece for detecting bilirubin by means of the diazo coupling reaction, comprising on a substrate a diazo compound selected from the group consisting of p-diazobenzene sulfonic acid, 2,6-dichlorobenzene diazonium tetrafluoroborate and 2-trifluoromethylbenzene diazonium tetrafluoroboric acid or 2,4-dichloroaniline and sodium nitrite which form a diazo compound and $NaIO_4$ in an amount of 0.9 to 15 mmol/m$^2$ based on said substrate.

2

DESCRIPTION OF THE PREFERRED EMBODIMENT

The test piece according to the present invention is designed to detect and measure bilirubin present in a given specimen by utilizing the diazo coupling reaction. For the test piece, while in use in the test described above, to avoid being affected by reducing substances such as ascorbic acid contained in the specimen under test, the oxidizing agent incorporated in the test piece is desirably $NaIO_4$.

In the test, the $NaIO_4$ is used in an amount large enough to give effective prevention of the inhibition of the reaction of an oxidizing indicator by reducing substances such as ascorbic acid present in the specimen under test. If the amount is less than $0.2$ $g/m^2$, the effect of the oxidizing agent is not manifested. If the amount is too much, the test piece is susceptible to deterioration by aging. The effective amount of the oxidizing agent is $0.9$ to $15$ $m.mol/m^2$ in terms of equivalent weight.

The diazo compound contained in the test piece according to the present invention is intended to react with bilirubin contained in the specimen under test to induce the diazo coupling with the decomposition product of bilirubin and give rise to a compound possessing a chromophore, azo group $-N=N-$. When the test piece is made to contain therein 2,4-dichloroaniline and sodium nitrite, a diazo compound capable of coupling with bilirubin is formed during the test by the reaction of 2,4-dichloroaniline with sodium nitrite. Thus, these compounds may be contained in the test piece in the place of the diazo compound.

Optionally, the test piece according to this invention may contain therein a buffer solution and a wetting agent. The buffer solution serves to maintain the pH value of the test piece immersed in the body fluid in the range of 4 to 8, preferably 5 to 7. Examples of the buffer solution usable for that purpose include citric acid-sodium citrate, tartaric acid-sodium tartarate, malic acid-borax, potassium phthalate-dipotassium phthalate, sodium hydrogen succinate and disodium succinate.

A wetting agent is used to enable the test piece immersed in the specimen to be uniformly wetted with the specimen. Examples of the wetting agent include surface active agents represented by alkyl sulfates such as sodium laurylsulfate, sodium dodecylsulfate and sodium tetradodecylsulfate, alkylbenzenesulfonates such as sodium dodecylbenzenesulfonate, cetyl pyridinium chloride and sodium diheptylsulfosuccinate.

The test piece obtained by impregnating the substrate with chemical agents may use therein a viscous agent adapted to prevent the contained chemical agents from exuding. Typical examples of the viscous agent include polymers such as polyvinyl alcohol, polyvinyl pyrrolidone, polyethylene glycol, polyacrylates, polyacrylamides, poly(hydroxyethyl methacrylate), poly(hydroxyethyl acrylate), carboxymethyl cellulose, and gelatin and gum arabic.

The use of the aforementioned chemical agents in the test piece of this invention is accomplished by dissolving these chemical agents in a solvent such as, for example, benzene, toluene, xylene methanol, ethanol, isopropanol, acetane, methylethyl ketone, chloroform, carbon tetrachloride or water, impregnating the substrate with the resultant solution, and drying the substrate wet with the chemical agents.

Examples of the substrate include filter paper and other similar forms of paper, non-woven fabrics formed of glass fibers or plastic material, and water-absorbing plastic sheets. Any of these substrates may be effectively used on the condition that it is incapable of reacting with the impregnating solution, insoluble in the solution, and capable of absorbing the solution. Paper is a preferred example.

After the test piece is produced by being impregnated with the solution of the chemical agents and subsequently dried, a thin peelable film of polyethylene, polyvinyl chloride, polyvinylidene chloride, or nylon may be applied on the test piece optionally for the purpose of protecting the test piece against deterioration of quality by humidity or ambient gas during the storage or service of the test piece and protecting the test piece against damage. This protective film is separated from the test piece immediately before the test piece is put to use.

Now, the present invention will be described more specifically below with reference to working examples.

Example 1

```
solution A:  p-Diazobenzene sulfonic acid    300 mg
             Metaphosphoric acid              15 g
             NaIO₄                           100 mg
             Water                           100 ml
Solution B:  Cetyl pyridinium chloride        2.5 g
             Ethanol                         100 ml
```

A filter pater was impregnated with Solution A and dried. Then it was impregnated with Solution B and dried. In specimens prepared by incorporating 50 mg/dl of ascorbic acid respectively in urine containing no bilirubin and urine containing bilirubin in a concentration of 0.5 mg/dl, samples of the test piece obtained as described above were immersed for test. The results were as shown in Table 1.

Example 2

```
solution A:  p-Diazobenzene sulfonic acid    300 mg
             Metaphosphoric acid              15 g
             CuSO₄                           100 mg


             Water                           100 ml
Solution B:  Cetyl pyridinium chloride        2.5 g
             Ethanol                         100 ml
```

A filter paper was impregnated with Solution A and dried. Then it was impregnated with Solution B and dried. In specimens prepared by incorporating 50 mg/dl of ascorbic acid respectively in urine containing no bilirubin and urine containing bilirubin in a concentration of 0.5 mg/dl, samples of the test piece obtained as described above were immersed for test. The resutls were as shown in Table 1.

Control 1

A test piece was produced by following the procedure of Example 1, except that $NaIO_4$ was omitted from Solution A. The results were as shown in Table 1.

EP 0 160 239 B1

## Table 1

| | Oxidizing agent | Urine containing ascorbic acid in a concentration of 50 mg/dl and no bilirubin | Urine containing ascorbic acid in a concentration of 50 mg/dl and bilirubin in a concentration of 0.5 mg/dl |
|---|---|---|---|
| Example 1 | $NaIO_4$ | − | + |
| Example 2 | $CuSO_4$ | − | + |
| Control 1 | − | − | − |

Note: + Coloration observed.
− Absence of coloration.

As described above, this invention relates to a test piece used for detection and determination of bilirubin by virtue of the diazo coupling reaction, which test piece is characterized by having supported on a substrate an effective amount of $NaIO_4$ in corporation with a diazo compound. Even when a given specimen such as urine happens to contain reducing substances such as asbcorbic acid, the diazo coupling reaction the test piece produces in the specimen is not impeded because the reducing substances are oxidized by the $NaIO_4$. The determination of bilirubin by the test piece of this invention is thus excellent when the diazo compound is selected from the group consisting of p-diazobenzene sulfonic acid, 2,6-dichlorobenzene diazonium tetrafluoroborates and 2-trifluoromethylbenzene diazonium tetrafluoroborates or the diazo compound is incorporated in the test piece by causing 2,4-dichloroaniline to react with sodium nitrite on the test piece.

## Claims

1. A test piece for detecting bilirubin by means of the diazo coupling reaction, comprising, on a substrate, a diazo compound selected from the group consisting of p-diazobenzene sulfonic acid, 2,6-dichlorobenzene diazonium tetrafluoroborate and 2-trifluoromethylbenzene diazonium tetrafluoroboric acid or 2,4-dichloroaniline and sodium nitrite which form a diazo compound and $NaIO_4$ in an amount of 0.9 to 15 mmol/m² based on said substrate.

2. A test piece according to claim 1, wherein said substrate is selected from the group consisting of paper, non-woven fabric of glass fibers or plastic material and water-absorbing plastic sheet.

3. A test piece according to claim 2, wherein said substrate is paper.

## Revendications

1. Elément analytique pour la détection de la bilirubine par la réaction de copulation avec un diazoïque, comprenant, sur un substrat, un composé diazoïque choisi dans le groupe constitué par l'acide p-diazobenzènesulfonique, un 2,6-dichlorobenzène diazonium tétrafluoroborate et l'acide 2-trifluorométhylbenzène diazonium tétrafluoroborique, ou la 2,4-dichloroaniline et le nitrite de sodium qui forment un composé diazoïque, et du $NaIO_4$ en une quantité de 0,9 à 15 mmol/m² relativement audit substrat.

2. Elément analytique selon la revendication 1, dans lequel ledit substrat est choisi dans le groupe constitué par le papier, un non-tissé de fibres de verre ou de matière plastique et une feuille de plastique absorbant l'eau.

3. Elément analytique selon la revendication 2, dans lequel ledit substrat est un papier.

5

**Patentansprüche**

1. Teststück zum Nachweis von Bilirubin mittels der Diazokupplungsreaktion, das auf einem Substrat eine Diazoverbindung aus der Gruppe p-Diazobenzolsulfonsäure, 2,6-Dichlorbenzoldiazoniumtetrafluorborat und 2-Trifluormethylbenzoldiazoniumtetrafluorborsäure oder 2,4-Dichloranilin und Natriumnitrit, die eine DiazoVerbindung bilden, und, bezogen auf das Substrat, 0,9 - 15 mMol/m$^2$ NaIO$_4$ enthält.

2. Teststück nach Anspruch 1, dadurch gekennzeichnet, daß das Substrat aus der Gruppe Papier, Gespinst oder Gewirk aus Glasfasern oder Kunststoffmaterial und wasserabsorbierende Kunststoffolie ausgewählt ist.

3. Teststück nach Anspruch 2, dadurch gekennzeichnet, daß das Substrat aus Papier besteht.